(19) Europäisches Patentamt
European Patent Office
Office européen des brevets

(11) **EP 1 967 138 A1**

(12) **EUROPEAN PATENT APPLICATION**

(43) Date of publication:
**10.09.2008 Bulletin 2008/37**

(51) Int Cl.:
***A61B 5/083*** *(2006.01)*

(21) Application number: **08004086.8**

(22) Date of filing: **05.03.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventors:<br>• **Chang, Keun-Shik**<br>  **Daejeon 305-701 (KR)**<br>• **Bae, Hwang**<br>  **Daejeon 305-701 (KR)** |
| (30) Priority: **05.03.2007 KR 20070021264** | (74) Representative: **Becker, Eberhard**<br>**Patentanwälte**<br>**Becker, Kurig, Straus**<br>**Bavariastrasse 7**<br>**80336 München (DE)** |
| (71) Applicant: **Korea Advanced Institute of Science and Technology**<br>**Daejeon, 305-701 (KR)** | |

(54) **Methodology and display instrument to noninvasively determine pulmonary characteristics through breath analysis and arterial blood measurement**

(57) Disclosed are a method for determining respiratory characteristics of lung-pulmonary circulation system by respiratory blood gas and blood gas data, and a displaying instrument for the same. More particularly, the present invention describes a method for determining respiratory characteristics of the lung-pulmonary circulation system by using respiratory blood gas and blood gas data and a display instrument for the same, so that the present invention provides partial $O_2$ and/or $CO_2$ pressure in other major parts, as well as shunt ratio of lungs and physiological dead space ratio by locally applying partial $O_2$ and/or $CO_2$ pressure information in lung-pulmonary circulation system in which gas-exchange is performed, and offers medical information such as cardiac output of a heart per beat.

Fig. 6

**Description**

BACKGROUND OF THE INVENTION

This application claims priority to Korean Patent Application No. 10-2007-0021264, filed on March 5, 2007, in the Korean Intellectual Property Office, the entire contents of which are hereby incorporated by reference.

1. Field of the Invention

The present invention relates to methodology and display instruments to non-invasively determine pulmonary blood gas characteristics; more particularly, the method is based on measurement of inspiration capacity $V_I$ (or $W_I$), $O_2$-$CO_2$ partial pressures of inspiration gas, I*, $O_2$-$CO_2$ partial pressures of end-tidal gas of breath, ET*, and $O_2$-$CO_2$ gas partial pressures of arterial blood, a*; the method makes use of these measured values as input to a computer program to determine $O_2$-$CO_2$ partial pressures of mixed venous, V*, $O_2$-$CO_2$ partial pressures of alveolar gas, A*, and respiratory characteristics of lungs-pulmonary circulation system such as $O_2$-$CO_2$ partial pressures of end-capillary blood, C*, pulmonary ventilation, VA*, end-capillary gas flow rate, Q, cardiac output, $Q_{total}$, shunt ratio Y, respiratory dead space ratio X, etc. Also, the present invention relates to an instrument to display the input values of measured breath and blood gas partial pressures and the output values of the pulmonary characteristics, wherein the measurement device for the input variables is connected either by a cable or wireless to the display device for the output variables such as computer terminal, printer, PDA, cellular phone and the like, so as to utilize the displayed output results for medical and health care purpose.

2. Description of the Related Art

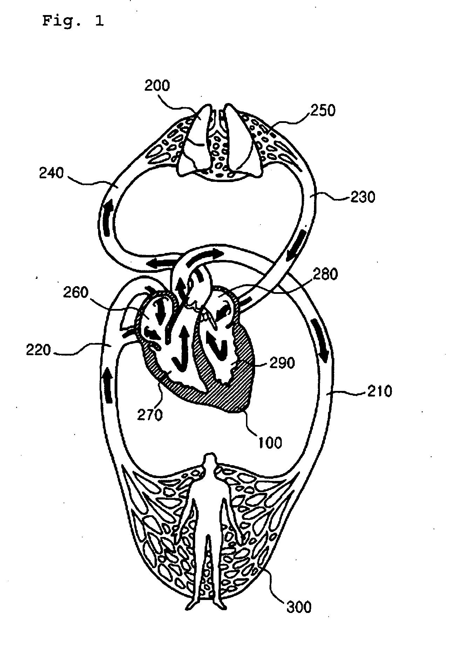

In general, the heartbeat of an adult in relaxed condition is about 60 to 70 times per minute. It is approximately equivalent to 100,000 per day or 2.6 billion times in a lifetime of 70 years. Blood volume in a body is about 5 liters and cardiac output per heartbeat is about 60 to 70cc; cardiac pumping rate is about 3.5 to 5.0 liters per minute; complete circulation of blood in a body takes about 40 to 50 seconds. Mechanical power of the heart is about 6,000cal per hour. A heart has two pumps, the right ventricle in charge of pulmonary circulation and the left ventricle in charge of systemic circulation. The vasculature includes aorta, pulmonary artery, coronary artery, artery, arteriole, end-capillary blood vessel, venules, vein, pulmonary vein, inferior and superior vena cavae. The extended total length of the blood vessel is about 96,000km, two and half times of the earth equator. Electricity is generated from the sinus node of heart and distributed by Purkinje fibers to make the heart muscles contract. Fig. 1 shows the order of blood circulation: right ventricle 270 → (pulmonary valve) → pulmonary artery 240 → pulmonary end-capillaries 250 → pulmonary vein 230 → left atrium 280 → (mitral valve) → left ventricle 290 → (aortic valve) → aorta 210 → artery → systemic end-capillaries 300 → vein → vena cava 220 → right atrium 260 → (tricuspid valve) →right ventricle 270.

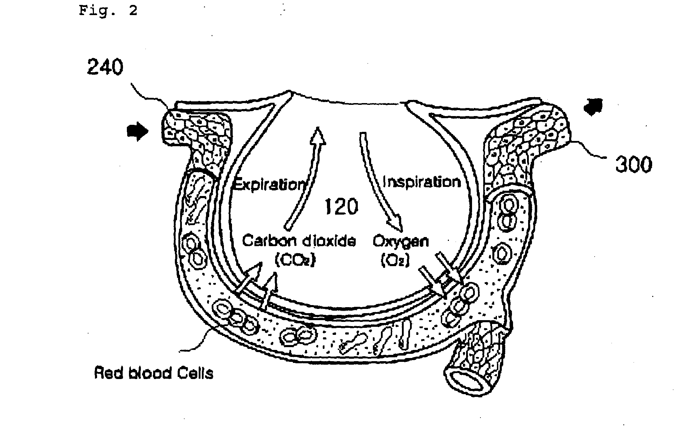

In the pulmonary circulation, blood is pumped by the right ventricle 270 to a network of a few micrometer-thick capillaries 250 via pulmonary arteries 240 in the right and left lungs 200; the blood is returned to the left atrium 280 via pulmonary vein 230, with very rapid two-way oxygen and carbon dioxide diffusion between breath air and blood through thin alveolar membrane of the capillaries, as shown in Fig. 2. Pulmonary arterial blood or the mixed venous blood is rich of carbon dioxide; pulmonary venous blood is rich of oxygen and becomes the arterial blood after it is pumped to the aorta by the left ventricle.

Gas exchange in the lung removes carbon dioxide and supply oxygen to the blood. Since there is no oxygen reservoir in the human body, one would die in a few minutes if oxygen supply is stopped by accident. Partial pressure of the $O_2$ and $CO_2$ blood gases of a patient is therefore very important information to a clinical doctor. Blood gas partial pressures can be measured using blood samples taken from the patient. The pulmonary vein is, however, hidden deep in the body to make the invasive mixed-venous blood sampling extremely difficult; it is not only painful but also costly. Therefore, it will be valuable if the partial pressures of the mixed-venous blood gas can be determined by mathematical and physiological model using input data obtained by minimally invasive method, such as $O_2$ and $CO_2$ partial pressures measured from arterial blood and breath gas; arterial blood can be sampled relatively easy and fast.

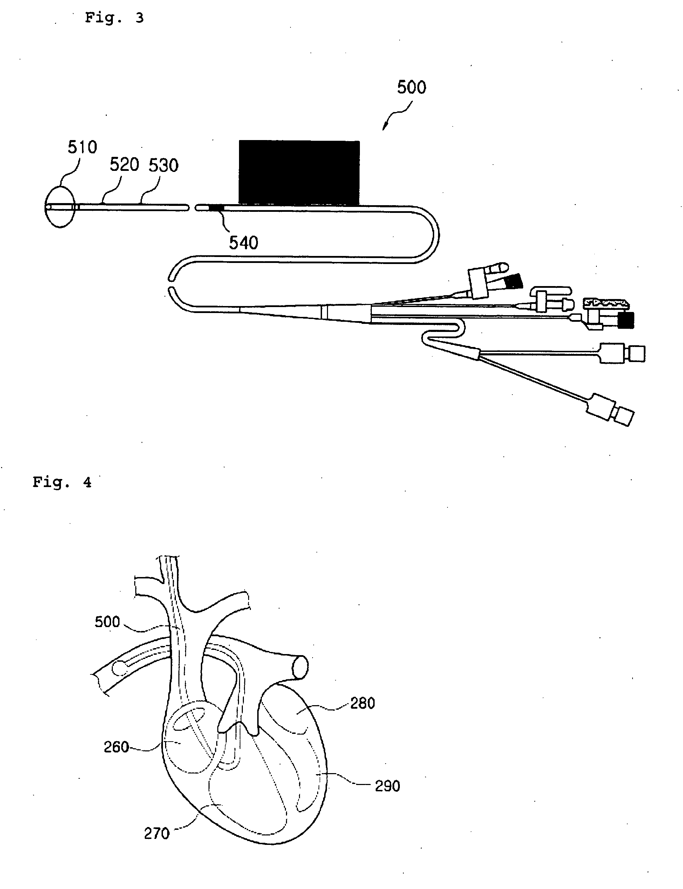

The first method, Korean Patent Application No. 1987-0002027, entitled "catheter for determining cardiac output and catheter for determining blood flow rate", disclosed a method of predicting the cardiac output or the blood volume flow rate pumped by the right ventricle; it is a direct measurement method using a catheter shown in Fig.3. The catheter 500 comprises: an opening 510 to discharge liquid and a temperature detector 530 or a thermistor 520 to determine the blood temperature at a distance from the opening; and a flow rate detector 540 installed near the thermistor 520. The flow rate detector 540 makes use of a self-heating thermistor and determines the blood volume flow rate by thermodilution principle.

The catheter is inserted into the pulmonary artery from the incision made on either jugular vein or femoral vein;

it passes through superior vena cava or inferior vena cava, right atrium 260 and right ventricle 270 as in Fig.4; a hot or cold liquid is injected into the right atrium 260 of heart and the thermistor measures how much the liquid is cooled or heated in the pulmonary artery.

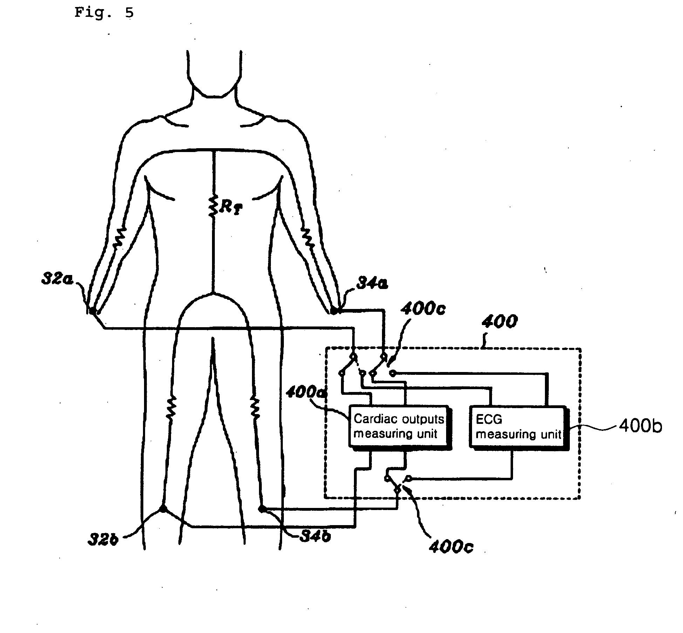

The second method, Korean Patent Application No. 10-1999-0000417, entitled "Method of attaching electrodes for monitoring ECG and cardiac outputs and apparatus of using the same", disclosed a method of evaluating cardiac output by electric signals from multiple electrodes mounted on the wrists (or ankles) or arms (or legs). Fig. 5. illustrates the apparatus comprising; current electrodes 32a and 32b mounted on the right hand (or arm) and right foot (or leg); voltage electrodes 34a and 34b mounted on the left hand (or arm) and left foot (or leg); a switching device 400c to connect the electrodes 32a and 32b, 34a and 34b to output devices such as cardiac output device 400a and electrocardiograph (ECG) 400b. The cardiac output device applies high frequency current to the current electrodes 32a and 32b through the switching device 400c; it measures voltages from the voltage electrodes 34a and 34b; it measures ECG by receiving difference signals from the voltage electrodes 34a and 34b; a control device outputs control signals to the switching device 400c to display the measured values on the display devices.

The third method is the so-called NICO (Non Invasive Cardiac Output); it employs a non-invasive evaluation of cardiac output by measuring expiration gas based on the *Partial $CO_2$ rebreathing method* developed by Novametrix Medical Systems. This method comprises measurement of $CO_2$ partial pressure in the expiration gas to solve Fick's equation for $CO_2$ to evaluate cardiac output. The above method is simple since it requires $CO_2$ input values with no need of other information like $O_2$; it, however, has a poor prediction accuracy.

The fourth method is Korean Patent Laid-Open No. 1999-22493; it disclosed a method of evaluating $O_2$ partial pressure in the mixed venous blood by solving Fick's equation using input values such as measured cardiac output, $O_2$ uptake and/or $O_2$ partial pressure in the arterial blood.

The first method has high accuracy but is a severely invasive method; it can possibly cause pain, complications and infection in the patients. The second method is inconvenient by the troubles of mounting electrodes on desired sites of human body. The third method is simple by using only the measured $CO_2$ partial pressure to solve Fick's equation. However, it is well known that this method has poor accuracy unless the cardiac output is about 6 liters/min. Likewise, the fourth method has poor accuracy because it makes use of insufficient number of input values and only one Fick's equation instead of many; the method is restricted because only $O_2$ partial pressure of mixed venous blood is predicted, not its $CO_2$ partial pressure.

In contrast, the present invention employs a more complete set of governing equations consisting of mass balance equations for $O_2$, $CO_2$ and $N_2$, shunt ratio equation, respiration quotient ratio equation, ventilation-perfusion ratio equation, etc.; it calculate as output the shunt ratio, dead space ratio, $O_2$-$CO_2$ partial pressures of alveolar gas, cardiac output and $O_2$-$CO_2$ partial pressure of mixed venous blood. The present invention is minimally invasive since the most-invasive $O_2$-$CO_2$ partial pressure of mixed venous blood is predicted, not measured. Therefore this method is clearly different from the conventional methods.

A human lung has some shunt and dead space even for a healthy person. A patient with respiratory disease has severe shunt and physiological dead space causing respiratory function to be significantly reduced. The present invention offers a method to determine shunt ratio, dead space ratio, $O_2$-$CO_2$ partial pressures of alveolar gas, alveolar ventilation, cardiac output, respiration quotient ratio; it applies a variety of more complex numerical formulae such as mass balance equation for $O_2$, mass balance equation for $CO_2$, shunt ratio equation for $O_2$, shunt ratio equation for $CO_2$, respiration quotient ratio equation for ventilation, respiration quotient ratio equation for blood, ventilation-perfusion ratio equation for $O_2$, ventilation-perfusion ratio equation for $CO_2$, etc.

A gas exchanging process in a lung means that blood simultaneously releases $CO_2$ and receives $O_2$ by diffusion, and must have a connection for reversing increase and decrease of partial pressures both of the gases. In general, $O_2$-$CO_2$ partial pressures of the alveolar gas A* is substantially equal to $O_2$-$CO_2$ partial pressures of end-capillaries C* through equilibrium at the end of expiration. If there is physiological dead space in a lung, non-functional air not used in gas exchange is mixed in the lung so that the end-tidal gas has lower $CO_2$ partial pressure and high $O_2$ partial pressure compared to functional air after completion of the gas exchange in the alveolar. If there is shunt in the lung, non-functional blood not used in gas exchange is mixed in the lung so that the arterial blood has lower $O_2$ partial pressure and high $CO_2$ partial pressure compared to functional blood after completion of the gas exchange in the capillary. Accordingly, measurement of the end-tidal gas or gas partial pressure of arterial blood cannot be connected by prediction of gas partial pressure for gas in alveolar and/or end-capillaries and collection of alveolar gas or blood in capillaries itself is very difficult, so that it is substantially impossible to find out $O_2$-$CO_2$ gas partial pressure of alveolar gas or blood in capillaries through direct measurement.

Consequently, using shunt or physiological dead space, it is very important to predict physiological characteristics of cardiopulmonary organs of a human body and there is a strong requirement for more advanced ideas and techniques to provide non-invasive and systematically rapid prediction means and/or instruments, compared to conventional methods known in the art.

SUMMARY OF THE INVENTION

Accordingly, the present invention has been proposed to solve problems such as damage at administration, side effects, uncertainty and/or delayed response in conventional techniques described above and an object of the present invention is to provide a method for computer analysis of numerical formulae systems such as mass balance equations for $O_2$ and $CO_2$, comprising: measuring flow rates of inspiration and/or expiration air in ventilation gas during breathing, and $O_2$-$CO_2$ partial pressure thereof; measuring $O_2$-$CO_2$ partial pressures of arterial blood as an input value; and using the input value to analyze the numerical formulae. The present invention further provides a method and an apparatus to accurately predict $O_2$-$CO_2$ partial pressures of mixed venous blood, $O_2$-$CO_2$ partial pressures of end-capillaries, $O_2$-$CO_2$ partial pressures of alveolar, cardiac output, shunt ratio and physiological dead space in respiratory organs by applying the solutions resulted from the computer analysis.

In order to accomplish the above described object, the present invention provides a method comprising the steps of: passing ventilation air through a nozzle; measuring inspiration capacity $V_I$ (or $W_I$), $O_2$-$CO_2$ partial pressures at inspiration I* and $O_2$-$CO_2$ partial pressures of end-tidal gas ET* through a gas sensor mounted on the nozzle during breathing and using the measured values as primary input values of respiratory gases; measuring $O_2$-$CO_2$ gas partial pressure of arterial blood a*; and inputting the measured values into a computer program and solving numerical systems such as mass balance equations for $O_2$ and $CO_2$ to determine significant physiological characteristics of the respiratory organs. The physiological characteristics are output values including, for example: respiratory functional characteristics such as $O_2$-$CO_2$ partial pressure of mixed venous blood and end-capillary (or commonly known as alveolar gas); cardiac functional characteristics such as cardiac output; and lung structural characteristics such as shunt ratio and physiological dead space. In addition, partial pressures and concentrations of $O_2$ and $CO_2$ which are combined with and/or separated from hemoglobin in red blood cells and diffused into blood and respiratory air reach an inter-equilibrium in alveolar and end-capillary, and are thereby capable of being inter-conversed through gas dissociation curve.

Primary measurement values entered as input data have less numerical values but require many respiratory characteristics to be determined.

Accordingly, the present invention can construct and analyze a system with more complex numerical formulae including, for example, mass balance equations for $O_2$ and $CO_2$, shunt ratio equations for $O_2$ and $CO_2$, respiratory equation of ventilation, respiratory equation of blood, ventilation-perfusion ratio equation for $O_2$ and $CO_2$ and the like, compared to those used in conventional methods.

BRIEF DESCRIPTION OF THE DRAWINGS

These and other objects, features, aspects, and advantages of the present invention will be more fully described in the following detailed description of preferred embodiments and examples, taken in conjunction with the accompanying drawings. In the drawings:

Fig. 1 is a schematic view illustrating order of blood circulation in heart and lungs of a human body;
Fig. 2 is a view describing alveolar model in which $O_2$ and $CO_2$ are diffused and exchanged with each other;
Fig. 3 illustrates a pulmonary arterial catheter for evaluating cardiac output by means of a conventional thermodilution method;
Fig. 4 is a view describing an example of catheter installation using a conventional right heart catheter;
Fig. 5 is a constructional view illustrating a conventional apparatus equipped with electrodes to display cardiac output and electrocardiogram (ECG);
Fig. 6 is a schematic view illustrating an apparatus to evaluate gas characteristics of alveolar gas and gas in end-capillary according to the present invention; and
Fig. 7 is graphs illustrating ventilation-perfusion ratio curves, input data and calculated prediction results determined by a method and an apparatus according to the present invention.

DETAILED DESCRIPTION OF THE INVENTION

Hereinafter, the present invention will be described in detail with reference to the accompanying drawings.

An aspect of the present invention is to adopt a systematic method to solve complicated formula systems described above, comprising: classifying respiratory problems into 'the first respiration model' with a small number of input values and 'the second respiration model' with a large number of input values; and applying analysis solutions and results induced from 'the first model' to a solution of 'the second model' as the more complicated question.

First, the first respiration model relates to a lung without shunt or physiological dead space, in which a blood boundary value comprises $O_2$-$CO_2$ partial pressures of mixed venous blood V* and a gas boundary value comprises $O_2$-$CO_2$ partial pressures of I* at the inspiration. The second respiration model relates to a lung with shunt or physiological dead space, in which a blood boundary value comprises information of mixed venous blood V*, supporting information for blood comprises $O_2$-$CO_2$ partial pressures of arterial blood a* and supporting information for gas comprises $O_2$-$CO_2$ partial pressures of end-tidal gas ET*. Also, the above respiration models have two types of models based on preference of variables in numerical solutions and, more particularly, the present invention classifies and describes 'first respiration model: first type model', 'first respiration model: second type model', 'second respiration model: first type model' and 'second respiration model: second type model'.

For 'first respiration model: first type model' as one of methods for determining respiratory characteristics of the present invention, the method includes the steps of: (a) inputting $O_2$-$CO_2$ partial pressure of mixed venous blood and $O_2$-$CO_2$ partial pressure of inspiration gas as specified boundary values, into an automatic computing device; (b) inputting an initial value VA/Q of ventilation-perfusion ratio for start of a do-loop into the automatic computing device; (c) inputting a pair of initial values of $O_2$-$CO_2$ partial pressure of alveolar gas, (A1, A2), for start of another do-loop, which satisfy ventilation-perfusion ratio equation, into the automatic computing device; (d) applying the $O_2$-$CO_2$ partial pressure of mixed venous blood, the $O_2$-$CO_2$ partial pressure of alveolar gas and the ventilation-perfusion ratio to solve the governing respiration equations; (e) solving a group of governing equations for respiratory blood gas in the automatic computing device and obtaining renewed values of alveolar-gas partial pressures (A1*, A2*) as a result; (f) calculating the renewed value VA/Q* for ventilation-perfusion ratio from the governing equations of respiratory gas, using the renewed $O_2$-$CO_2$ partial pressure of alveolar gas (A1*, A2*); (g) determining whether the renewed ventilation-perfusion ratio value VA/Q* satisfies the requirement of solution; (h) making decision whether the renewed $O_2$-$CO_2$ partial pressures (A1*, A2*) in pair with the renewed ventilation-perfusion ratio VA/Q* are correct solutions.

In the step (a), the $O_2$-$CO_2$ partial pressures of mixed venous blood V* and the $O_2$-$CO_2$ partial pressures of inspiration gas I* are directly measured or usually obtained from alternative sources.

In the step (e), the respiratory governing equations for respiratory blood gas comprises mass balance equations for $O_2$, $CO_2$ and $N_2$, combined equations for gas partial pressure such as Equation Nos. 3 to 6, and ventilation-perfusion ratio equations such as Equation Nos. 11 and 12. More particularly, $O_2$ partial pressure of alveolar A1* is obtained by substituting VA/Q (a initial value in the step (b)) for Eq. 3 as a mass balance equation for $O_2$, or for Eq. 11 as a ventilation-perfusion ratio equation for $O_2$. Likewise, $CO_2$ partial pressure of alveolar A2* is obtained by substituting VA/Q for Eq. 4 as a mass balance equation for $CO_2$, or for Eq. 12 as a ventilation-perfusion ratio equation for $CO_2$.

In the step (g), the ventilation-perfusion ratio requirement is characterized in directly determining whether a difference of the calculated initial value VA/Q for ventilation-perfusion ratio in the step (b) and the renewed value VA/Q* obtained in the step (f) is within a constant range and, if the requirement is not satisfied, the present inventive method includes returning to the step (c) and repeatedly carrying out the steps (d) to (g) for a renewed pair of $O_2$-$CO_2$ partial pressure of alveolar gas (A1, A2).

In the above step (g), the ventilation-perfusion ratio requirement is further characterized in indirectly determining whether a difference of the setup $O_2$-$CO_2$ partial pressure (A1, A2) inputted in the step (c) and the renewed $O_2$-$CO_2$ partial pressure (A1*, A2*) obtained in the step (e) are within constant ranges, respectively.

The renewed pair of $O_2$-$CO_2$ partial pressure of alveolar gas (A1, A2) in the step (c) comprise $CO_2$ partial pressure A2 renewed according to a specific regulation and the renewed $O_2$ partial pressure A1* obtained from the ventilation-perfusion ratio equation in association with the $CO_2$ partial pressure A2. Similarly, the pair of $O_2$-$CO_2$ partial pressure of alveolar gas (A1*, A2*) obtained in the step (e) comprise the renewed $O_2$ partial pressure A1* obtained in the step (c) and $CO_2$ partial pressure A2* obtained by solving a group of governing equations for respiratory blood gas using the renewed $O_2$ partial pressure A1*.

The renewed pair of $O_2$-$CO_2$ partial pressure of alveolar gas (A1, A2) in the step (c) comprise $O_2$ partial pressure A1 renewed according to a specific regulation and the renewed $CO_2$ partial pressure A2* obtained from the ventilation-perfusion ratio equation in association with the $O_2$ partial pressure A1. Similarly, the pair of $O_2$-$CO_2$ partial pressure of alveolar gas (A1*, A2*) obtained in the step (e) comprise the renewed $CO_2$ partial pressure A2* obtained in the step (c) and $O_2$ partial pressure A1* obtained by solving a group of governing equations for respiratory blood gas using the renewed $CO_2$ partial pressure A2*.

The pair of $O_2$-$CO_2$ partial pressure of alveolar gas (A1*, A2*) obtained in the step (h) comprise solutions (A1*, A2*) obtained from the steps (c) to (e) using initial value VA/Q and renewed value VA/Q* for ventilation-perfusion ratio in the step (b).

A method for determining respiratory characteristics includes the steps of: obtaining a solution A* of $O_2$-$CO_2$ partial pressures of alveolar gas in the step (h) corresponding to initial value VA/Q for ventilation-perfusion ratio in the step (b); renewing initial value VA/Q for ventilation-perfusion ratio after returning to the step (b); and repeatedly carrying out the steps (c) to (h).

Also, a method for determining respiratory characteristics includes repeating the steps (a) to (h) to obtain a desired distribution of $O_2$-$CO_2$ partial pressures A* of alveolar gas corresponding to all of initial values VA/Q for ventilation-perfusion ratio.

Requirement for ventilation-perfusion ratio in the step (h) means ventilation-perfusion ratio is within a specific range, which is calculated by any of various ventilation-perfusion ratio equations including ventilation-perfusion ratio equation (Eq. 9) induced from Fick's equation for $O_2$ (Eq. 1); ventilation-perfusion ratio equation (Eq. 11) induced from mass balance equation for $O_2$ (Eq. 3); ventilation-perfusion ratio equation (Eq. 10) induced from Fick's equation for $CO_2$ (Eq. 2); and/or ventilation-perfusion ratio equation (Eq. 12) induced from mass balance equation for $CO_2$ (Eq. 4). The respiratory characteristics are determined when the above requirement is satisfied.

The first respiration model for determining respiratory characteristics according to the present invention is solved by means of ventilation-perfusion ratio curve or Kelman's curve. Especially, with regard to 'first respiration model: first type model', an computing subroutines for determining respiratory characteristics and analysis thereof is described as follows.

At first, the computing subroutines is run in a computer containing a computer program written by the present inventors and a principal construction for analysis of the above determining method includes the following loops and/or steps of: forming an outer do-loop and inputting an initial value VA/Q of ventilation-perfusion ratio into the loop; forming an inner do-loop and inputting an initial value of $O_2$ partial pressure information A1 of alveolar gas (referring to as A1*); calculating $CO_2$ partial pressure A2* by solving a mass balance equation; and using a pair of the values A1* and A2* to calculate another value VA/Q* for ventilation-perfusion ratio. When the value VA/Q is equal to the value VA/Q* as the requirement for ventilation-perfusion ratio, A1* and A2* are defined as solutions to escape the inner do-loop. For renewal of initial value of VA/Q in the outer do-loop, the above calculation process is repeated to offer a Kelman's curve formed of a set of $O_2$-$CO_2$ partial pressure of alveolar matching A* regularly altered VA/Q values, which is often called ventilation-perfusion ratio curve or $O_2$-$CO_2$ diagram. As the above 'first respiration model' has no shunt or physiological dead space, the $O_2$-$CO_2$ partial pressures A* is substantially equal to any one selected from information of end-capillary blood C*, information of end-tidal gas ET* and $O_2$-$CO_2$ partial pressures of arterial blood a*.

Next, 'first respiration model: second type model' is a method for using a pair of $O_2$-$CO_2$ partial pressures initial values A1 and A2 of alveolar gas and comprises the steps of: (a) inputting $O_2$-$CO_2$ partial pressures of mixed venous blood and $O_2$-$CO_2$ partial pressure of inspiration gas as specified boundary values, into an automatic computing device; (b) inputting a pair of initial values of $O_2$-$CO_2$ partial pressure of alveolar gas, (A1, A2), for start of a do-loop into the automatic computing device; (c) inputting an initial value VA/Q for ventilation-perfusion ratio, for start of another do-loop, which satisfy ventilation-perfusion ratio equation using the above $O_2$-$CO_2$ partial pressures initial values (A1, A2), into the automatic computing device; (d) applying the $O_2$-$CO_2$ partial pressure of mixed venous blood, the $O_2$-$CO_2$ partial pressure of alveolar gas and the ventilation-perfusion ratio to solve the governing respiration equations; (e) solving a group of governing equations for respiratory blood gas in the automatic computing device and obtaining renewed values of alveolar-gas partial pressures (A1*, A2*) as a result; (f) calculating the renewed value VA/Q* for ventilation-perfusion ratio from the governing equations of respiratory gas, using the renewed $O_2$-$CO_2$ partial pressure of alveolar gas (A1*, A2*); (g) determining whether the renewed $O_2$-$CO_2$ partial pressure of alveolar gas (A1*, A2*) satisfies requirement of solution; and (h) making decision whether the renewed $O_2$-$CO_2$ partial pressures (A1*, A2*) in pair with the initial ventilation-perfusion ratio VA/Q are correct solutions.

In the step (a), the $O_2$-$CO_2$ partial pressures of mixed venous blood **V*** and the $O_2$-$CO_2$ partial pressures of inspiration gas **I*** in the step (a) are directly measured or usually obtained from alternative sources. In the step (e), the equation group for respiratory blood gas comprises mass balance equations for $O_2$, $CO_2$ and $N_2$, and combined equations for gas partial pressure.

In the step (g), the ventilation-perfusion ratio requirement is characterized in determining whether a difference of the calculated initial value VA/Q and the renewed value VA/Q* for ventilation-perfusion ratio obtained in the step (f) is within a constant range and, if the requirement is not satisfied, the present inventive method includes returning to the step (b) and repeatedly carrying out the steps (c) to (g) for a renewed pair of $O_2$-$CO_2$ partial pressure of alveolar gas A1 and A2.

The renewed pair of $O_2$-$CO_2$ partial pressure of alveolar gas A1 and A2 in the step (b) comprise $CO_2$ partial pressure A2 setup as an initial value and $O_2$ partial pressure A1 obtained by mass balance equation for $O_2$ using the $CO_2$ partial pressure A2. Similarly, the pair of $O_2$-$CO_2$ partial pressure of alveolar gas A1* and A2* obtained in the step (e) comprise $CO_2$ partial pressure A2 as an initial value (referring to as A2*) obtained in the step (b) and $O_2$ partial pressure A1* obtained by solving a group of governing equations for respiratory blood gas using the initial $CO_2$ partial pressure A2*.

The renewed pair of $O_2$-$CO_2$ partial pressure of alveolar gas A1 and A2 in the step (b) comprise $O_2$ partial pressure A1 setup as an initial value and $CO_2$ partial pressure A2 obtained by mass balance equation for $CO_2$ using the initial $O_2$ partial pressure A1. Similarly, the pair of $O_2$-$CO_2$ partial pressure of alveolar gas A1* and A2* obtained in the step (e) comprise $O_2$ partial pressure A1 as an initial value (referring to as A1*) obtained in the step (b) and $CO_2$ partial pressure A2* obtained by solving a group of governing equations for respiratory blood gas using the initial $O_2$ partial pressure A1*.

In the step (h), the ventilation-perfusion ratio requirement is characterized in directly determining whether a difference of the calculated initial value VA/Q for ventilation-perfusion ratio in the step (c) and the renewed value VA/Q* obtained in the step (f) is within a constant range and, if the requirement is not satisfied, the present inventive method includes returning to the step (b) and repeatedly carrying out the steps (c) to (h) for a renewed pair of $O_2$-$CO_2$ partial pressure of alveolar gas A1 and A2 .

In the above step (h), the ventilation-perfusion ratio requirement is further characterized in indirectly determining whether a difference of the setup $O_2$-$CO_2$ partial pressure A1 and A2 inputted in the step (b) and the renewed $O_2$-$CO_2$ partial pressure A1* and A2* obtained in the step (e) are within constant ranges, respectively.

A method for determining respiratory characteristics includes the steps of: obtaining final solutions of $O_2$-$CO_2$ partial pressure of alveolar gas (A1*, A2*) with regard to $O_2$-$CO_2$ partial pressures initial values of alveolar gas (A1, A2) or initial value VA/Q for ventilation-perfusion ratio corresponding thereto; renewing $O_2$ partial pressure A1 after returning to the step (b); and repeatedly carrying out the steps (c) to (h).

Also, a method for determining respiratory characteristics includes the steps of: obtaining final solutions of $O_2$-$CO_2$ partial pressure of alveolar gas (A1*, A2*) with regard to $O_2$-$CO_2$ partial pressures initial values of alveolar gas (A1, A2) or initial value VA/Q for ventilation-perfusion ratio corresponding thereto; renewing $CO_2$ partial pressure A2 after returning to the step (b); and repeatedly carrying out the steps (c) to (h).

However, in case of practically determining respiratory characteristics, shunt or physiological dead space must be considered. Therefore, 'second respiration model' is preferably suggested in the method for determining extended respiratory characteristics according to the present invention. Among "second respiration model', 'second respiration model: first type model' has physiological dead space ratio X as an initial value while 'second respiration model: second type model' has shunt ratio Y as an initial value.

Hereinafter, the present invention will be more particularly explained for the 'second respiration model: second type model' in the following description.

'Second respiration model: first type model' of the present invention comprises the steps of: (a) inputting blood boundary value, gas boundary value, supporting information for blood, supporting information for gas and inspiration flow rate into an automatic computing device; (b) inputting initial value of physiological dead space ratio X into the automatic computing device; (c) inputting a pair of $O_2$-$CO_2$ partial pressures initial values of alveolar gas (A1, A2) into the automatic computing device using the initial value of the dead space ratio X; (d) applying the boundary value, the initial value and the initial values to an computing subroutines built in the automatic computing device; (e) solving a group of governing equations for respiratory blood gas in the computing subroutines and obtaining newly renewed $O_2$-$CO_2$ partial pressure of alveolar gas (A1*, A2*) based on the solutions; (f) calculating $O_2$ shunt ratio Y1 and $CO_2$ shunt ratio Y2 if the renewed $O_2$-$CO_2$ partial pressure satisfy requirement for $O_2$-$CO_2$ partial pressures; (g) determining desired respiratory characteristics if the shunt ratio requirement is satisfied; and (h) determining cardiac output.

In the above step (a), the blood boundary value comprises $O_2$-$CO_2$ partial pressures V* of mixed venous blood or $O_2$ partial pressure only while the gas boundary value comprises all of $O_2$-$CO_2$ partial pressure at inspiration I* or $O_2$ partial pressure only. Also, the supporting information for blood comprises all of $O_2$-$CO_2$ partial pressure of arterial blood a* or $O_2$ partial pressure only.

In the above step (a), the supporting information for gas comprises all of $O_2$-$CO_2$ partial pressure of end-tidal gas ET* or $CO_2$ partial pressure only. Inspiration capacity VI in the step (a) means flow rate of external air entered into lungs and, for tidal breathing, the capacity VI is substantially equal to expiration capacity VE released outside from the lungs.

In the step (e), the equation group for respiratory blood gas comprises mass balance equations for $O_2$, $CO_2$ and $N_2$ and combined equations for gas partial pressure.

A method for determining respiratory characteristics is characterized by repetition of the steps (d) and (e) for a renewed pair of $O_2$-$CO_2$ partial pressures initial values of alveolar gas (A1, A2) after returning to the step (c) if the requirements for $O_2$-$CO_2$ partial pressures in the step (f) were not satisfied.

In the step (c), the $O_2$-$CO_2$ partial pressure (A1, A2) are a new pair of partial pressures (A1, A2*) comprising $O_2$ partial pressure A1 to be a repeatedly renewed initial value and $CO_2$ partial pressure A2 (referred to as A2*) obtained using the initial value of the dead space ratio X.

The renewed $O_2$-$CO_2$ partial pressure of alveolar gas (A1*, A2*) obtained in the step (e) comprise the $CO_2$ partial pressure of alveolar gas A2* obtained in the step (c) and renewed $O_2$ partial pressure A1* obtained by solving a group of governing equations for respiratory blood gas including mass balance equations for $O_2$, $CO_2$ and $N_2$ and combined equations for gas partial pressure using the $O_2$-$CO_2$ partial pressure (A1, A2*) determined in the step (c).

Requirement for $O_2$-$CO_2$ partial pressures in the step (f) is characterized in determining whether a difference between the $O_2$ partial pressure A1 renewed initial value in the step (c) and the $O_2$ partial pressure A1* obtained by solving a group of governing equations for respiratory blood gas is within a specific range.

In the step (c), the $O_2$-$CO_2$ partial pressure of alveolar gas (A1, A2) are a new pair of partial pressures (A1*, A2) comprising $CO_2$ partial pressure A2 to be a repeatedly renewed initial value and $O_2$ partial pressure A1 (referred to as A1*) obtained using the initial shunt ratio X.

The renewed $O_2$-$CO_2$ partial pressure of alveolar gas (A1*, A2*) obtained in the step (e) comprise the $O_2$ partial

pressure of alveolar gas A1* obtained in the step (c) and renewed $CO_2$ partial pressure A2* obtained by solving a group of governing equations for respiratory blood gas including mass balance equations for $O_2$, $CO_2$ and $N_2$ and combined equations for gas partial pressure using the $O_2$-$CO_2$ partial pressure (A1*, A2*) determined in the step (c).

Requirement for $O_2$-$CO_2$ partial pressures in the step (f) is characterized in determining whether a difference between the $CO_2$ partial pressure A2 renewed initial value in the step (c) and the $CO_2$ partial pressure A2* obtained by solving a group of governing equations for respiratory blood gas is within a specific range.

Requirement for shunt ratio in the step (g) is characterized in determining whether a difference between $O_2$ shunt ratio Y1 and $CO_2$ shunt ratio Y2 is within a specific range and, if the requirement for shunt ratio is not satisfied, the present inventive method includes returning to the step (b) and repeatedly renewing physiological dead space ratio X.

Briefly, the above 'second respiration model: first type model' comprises the steps of: setting up any greater value than $O_2$ partial pressure of arterial blood a1 as an initial value for $O_2$ partial pressure A1; and inputting an initial dead space ratio X and an initial value for $CO_2$ partial pressure A2 calculated using the dead space ratio X into an automatic computing device. By substituting the initial values for mass balance equation Eq. 4 or Eq. 12 for $CO_2$, a corresponding value VA/Q for ventilation-perfusion ratio is obtained. Also, the renewed $O_2$ partial pressure A1* is obtained by substituting the above initial values and the value VA/Q for mass balance equation Eq. 3 or Eq. 11 for $O_2$. When a difference between $O_2$ shunt ratio Y1 and $CO_2$ shunt ratio Y2 is not within a specific range, the present inventive method includes returning to the step (b), setting up a new dead space ratio X so as to calculate a new renewed $CO_2$ partial pressure A2*, and repeatedly carrying out the same calculation to obtain a new $O_2$ partial pressure of alveolar gas A1**. From the resulting values, alternative $O_2$ shunt ratio Y1* and $CO_2$ shunt ratio Y2* are calculated again and the same calculation is repeatedly carried out until a difference between these values Y1* and Y2* is within the specific range, thereby obtaining $O_2$-$CO_2$ partial pressures of alveolar gas.

Respiratory characteristics determined in the above step (g) include latest stored $O_2$-$CO_2$ partial pressure of alveolar gas (A1*, A2*) during repeat process, $O_2$-$CO_2$ partial pressures of end-capillary C*, shunt ratio Y1* or Y2* and physiological dead space ratio X*.

Cardiac output determined in the step (h) is obtained by equations Eq. 15 and 16 and using measured inspiration capacity VI or expiration capacity VE, perfusion capacity Q, shunt ratio Y*, and physiological dead space ratio X*.

In other words, the above 'second respiration model: first type model' uses information of mixed venous blood V* and information of arterial blood a* as blood input values as well as inspiration gas information I* and end-tidal gas information ET* as gas input values.

A method for analysis of the above model questions and an computing subroutines for the same is more particularly described as follows.

At first, the computing subroutines is run in a computer containing a computer program written by the present inventors, and a principal construction for analysis of the present inventive method for determining respiratory characteristics includes the following loops and/or steps of: forming an outer do-loop and setting up an initial value of dead space ratio X; inputting an initial value of $O_2$ partial pressure of alveolar gas A1 into an inner do-loop and calculating $CO_2$ partial pressure of alveolar gas A2 by solving a mass balance equation system; or, inputting an initial value of $CO_2$ partial pressure of alveolar gas A2 into an inner do-loop and calculating $O_2$ partial pressure of alveolar gas A1 by solving a mass balance equation system; using $O_2$-$CO_2$ partial pressures setup data of alveolar gas (A1, A2) to calculate $O_2$ shunt ratio Y1 by an equation Eq. 7 and calculate shunt ratio Y2 by an equation Eq. 8, and determining whether these values are same as each other as a requirement for shunt ratio; if the result does not satisfy the requirement for shunt ratio, returning to the start of outer do-loop to renew the initial value for dead space ratio X and repeating the above calculation; if the result satisfies the requirement for shunt ratio, releasing out of both of the inner do-loop and the outer do-loop and taking $O_2$-$CO_2$ partial pressures of alveolar gas A*, shunt ratio Y* and physiological dead space ratio X* from the finally stored values in a memory device as final solutions; and applying inputted inspiration capacity VI (or $W_I$ ), calculated perfusion Q, dead space ratio X* and shunt ratio Y* to determine cardiac output $Q_{total}$ according to a cardiac output equation Eq. 15 or 16.

Meanwhile, an alternative method for determining respiratory characteristics of the present invention is 'second respiration model: second type model', comprising the steps of: (a) inputting blood boundary value, gas boundary value, supporting information for blood, supporting information for gas and inspiration capacity into an automatic computing device; (b) inputting an initial value of shunt ratio Y into the automatic computing device; (c) inputting a pair of $O_2$-$CO_2$ partial pressures initial values of alveolar gas (A1, A2) obtained by the initial shunt ratio Y into the automatic computing device; (d) applying the boundary value, the initial value and the initial values to an computing subroutines built in the automatic computing device; (e) solving a group of governing equations for respiratory blood gas in the computing subroutines and obtaining renewed $O_2$-$CO_2$ partial pressure of alveolar gas (A1*, A2*) based on the solutions of the equation group; (f) calculating $O_2$ dead space ratio X1 and $CO_2$ dead space ratio X2 if the renewed $O_2$-$CO_2$ partial pressure (A1*, A2*) satisfy requirements for $O_2$-$CO_2$ partial pressures; (g) determining desired respiratory characteristics if the dead space ratio requirement is satisfied; and (h) determining cardiac output.

In the above step (a), the gas boundary value comprises all of $O_2$-$CO_2$ partial pressure at inspiration I* or $O_2$

partial pressure only. Also, the supporting information for blood comprises all of $O_2$-$CO_2$ partial pressure of arterial blood a* or $O_2$ partial pressure only.

In the above step (a), the supporting information for gas comprises all of $O_2$-$CO_2$ partial pressure of end-tidal gas ET* or $CO_2$ partial pressure only. Inspiration capacity VI in the step (a) means flow rate of external air entered into lungs and, for tidal breathing, the capacity VI is substantially equal to expiration capacity VE released outside from the lungs.

In the step (e), the analysis equation group for respiratory blood gas comprises mass balance equations for $O_2$, $CO_2$ and $N_2$ and combined equations for gas partial pressure.

A method for predicting respiratory characteristics is characterized by repetition of the steps (d) and (e) for a renewed pair of $O_2$-$CO_2$ partial pressures initial values of alveolar gas (A1, A2) after returning to the step (c) if the requirements for $O_2$-$CO_2$ partial pressures in the step (f) were not satisfied.

In the step (c), the $O_2$-$CO_2$ partial pressure of alveolar gas (A1, A2) are a new pair of partial pressures (A1, A2*) comprising $O_2$ partial pressure A1 to be a repeatedly renewed initial value and $CO_2$ partial pressure A2 (referred to as A2*) obtained using the initial shunt ratio Y.

The renewed $O_2$-$CO_2$ partial pressure of alveolar gas (A1*, A2*) obtained in the step (e) comprise the $CO_2$ partial pressure of alveolar gas A2* obtained in the step (c) and renewed $O_2$ partial pressure A1* obtained by solving a group of governing equations for respiratory blood gas including mass balance equations for $O_2$, $CO_2$ and $N_2$ and combined equations for gas partial pressure using the $O_2$-$CO_2$ partial pressure (A1, A2*) determined in the step (c).

Requirement for $O_2$-$CO_2$ partial pressures in the step (f) is characterized in determining whether a difference between the $O_2$ partial pressure A1 renewed initial value in the step (c) and the $O_2$ partial pressure A1* obtained by solving a group of governing equations for respiratory blood gas is within a specific range.

In the step (c), the $O_2$-$CO_2$ partial pressure (A1, A2) are a new pair of partial pressures (A1*, A2) comprising $CO_2$ partial pressure A2 to be a repeatedly renewed initial value and $O_2$ partial pressure A1 (referred to as A1*) obtained using the initial shunt ratio Y.

The renewed $O_2$-$CO_2$ partial pressure of alveolar gas (A1*, A2*) obtained in the step (e) comprise the $O_2$ partial pressure of alveolar gas A1* obtained in the step (c) and renewed $CO_2$ partial pressure A2* obtained by solving a group of governing equations for respiratory blood gas including mass balance equations for $O_2$, $CO_2$ and $N_2$ and combined equations for gas partial pressure using the $O_2$-$CO_2$ partial pressure (A1*, A2) determined in the step (c).

Requirement for $O_2$-$CO_2$ partial pressures in the step (f) is characterized in determining whether a difference between the $CO_2$ partial pressure A2 renewed initial value in the step (c) and the $CO_2$ partial pressure A2* obtained by solving a group of governing equations for respiratory blood gas is within a specific range.

Requirement for shunt ratio in the step (g) is characterized in determining whether a difference between $O_2$ dead space ratio X1 and $CO_2$ dead space ratio X2 is within a specific range and, if the requirement for dead space ratio is not satisfied, the present inventive method includes returning to the step (b) and repeatedly renewing physiological shunt ratio Y.

Briefly, the above 'second respiration model: second type model' comprises the steps of: setting up any greater value than $O_2$ partial pressure of arterial blood a1 as an initial value for $O_2$ partial pressure A1; and inputting an initial shunt ratio Y and an initial value for $CO_2$ partial pressure A2 calculated using the shunt ratio Y into an automatic computing device. By substituting the initial values for mass balance equation Eq. 4 or Eq. 12 for $CO_2$, a corresponding value VA/Q for ventilation-perfusion ratio is obtained. Also, the renewed $O_2$ partial pressure A1* is obtained by substituting the above initial values and the value VA/Q for mass balance equation Eq. 3 or Eq. 11 for $O_2$. When a difference between $O_2$ dead space ratio X1 and $CO_2$ dead space ratio X2 is not within a specific range, the present inventive method includes returning to the step (b), setting up a new shunt ratio Y so as to calculate a new renewed $CO_2$ partial pressure A2*, and repeatedly carrying out the same calculation to obtain a new $O_2$ partial pressure of alveolar gas A1**. From the resulting values, alternative $O_2$ dead space ratio X1* and $CO_2$ dead space ratio X2* are calculated again and the same calculation is repeatedly carried out until a difference between these values is within the specific range, thereby obtaining $O_2$-$CO_2$ partial pressures of alveolar gas.

Respiratory characteristics determined in the above step (g) include latest stored $O_2$-$CO_2$ partial pressure of alveolar gas (A1*, A2*) during repeat process, $O_2$-$CO_2$ partial pressures of end-capillary C*, physiological dead space ratio X1* or X2* and shunt ratio Y*.

Cardiac output determined in the step (h) is obtained by equations Eq. 15 and 16 and using measured inspiration capacity VI or expiration capacity VE, perfusion capacity Q, shunt ratio Y*, and physiological dead space ratio X*.

In other words, the above 'second respiration model: second type model' uses information of mixed venous blood V* and information of arterial blood a* as blood input values as well as inspiration gas information I* and end-tidal gas information ET* as gas input values.

A method for analysis of the above model questions and an computing subroutines for the same is more particularly described as follows.

At first, the computing subroutines is run in a computer containing a computer program written by the present inventors, and a principal construction for analysis of the present inventive method for determining respiratory characteristics includes the following loops and/or steps of: forming an outer do-loop and setting up an initial value of shunt ratio Y; inputting an initial value of $O_2$ partial pressure of alveolar gas A1 into an inner do-loop and calculating $CO_2$ partial pressure of alveolar gas A2 by solving a mass balance equation system; or, inputting an initial value of $CO_2$ partial pressure of alveolar gas A2 into an inner do-loop and calculating $O_2$ partial pressure of alveolar gas A1 by solving a mass balance equation system; using $O_2$-$CO_2$ partial pressures setup data of alveolar gas (A1, A2) to calculate $O_2$ dead space ratio X1 by an equation Eq. 17 and calculate $CO_2$ dead space ratio X2 by an equation Eq. 18, and determining whether these values are same as each other as a requirement for dead space ratio; if the result does not satisfy the requirement for shunt ratio, returning to the start of outer do-loop to renew the initial value for shunt ratio Y and repeating the above calculation; if the result satisfies the requirement for dead space ratio, releasing out of both of the inner do-loop and the outer do-loop and taking $O_2$-$CO_2$ partial pressures of alveolar gas A*, shunt ratio Y* and physiological dead space ratio X* from the finally stored values in a memory device as final solutions; and applying inputted inspiration capacity VI (or $W_I$), calculated perfusion Q, dead space ratio X* and shunt ratio Y* to determine cardiac output $Q_{total}$ according to a cardiac output equation Eq. 15 or 16.

Furthermore, an instrument for displaying respiratory characteristics according to the present invention includes an information terminal connected to the automatic computing device to visually display results predicted by the method for determining respiratory characteristics of the present invention, so as to preferably and conveniently provide the results to a user. Such computing device may comprise computer processors and/or embedded chips fixed in a computer.

The present inventive display instrument is portably carried by wired or wireless connection of the information terminal to the computing device.

As described above, the display instrument is a system for simultaneously predicting various physiological characteristics of cardiopulmonary organs including, for example: respiratory functional characteristics such as $O_2$-$CO_2$ partial pressure of mixed venous blood and end-capillary (or commonly known as alveolar gas); cardiac functional characteristics such as cardiac output; and lung structural characteristics such as shunt ratio and physiological dead space ratio. The instrument comprises: a nozzle 1 for passing ventilation air; means 3, 4 and 5 for measuring primary variables in ventilation through a gas sensor mounted on the ventilation nozzle 1; a means for measuring $O_2$-$CO_2$ partial pressures of arterial blood; a means 7 for analyzing numerical formulae systems such as mass balance equations for $O_2$ and $CO_2$ by computer to induce blood respiratory characteristics, cardiac functional characteristics and lung structural characteristics; and a display means 8 for visually illustrating various input and output values.

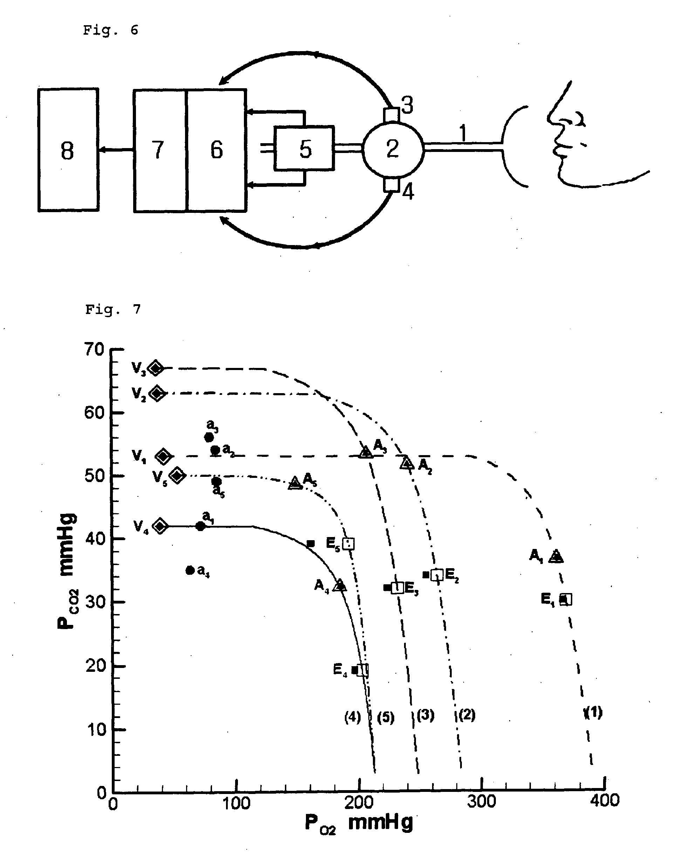

Fig. 6 is a systematic view to illustrate an instrument for determining respiratory characteristics of lung-pulmonary circulation system according to the present invention. Such instrument includes: a mask and a nozzle 1 and 2 for passing ventilation air; sensor measuring means 3, 4 and 5 to measure primary respiratory values of the ventilation air from sensors mounted on the ventilation nozzle; a micro-processor or computer 7 which has a program to solve respiration model questions in order to calculate respiratory characteristics of lung-pulmonary circulation system by using the primary respiratory values; and a means 8 for visually displaying the primary values and renewed respiratory characteristics on liquid crystal displays, computer terminals, printers, mobile phones, PDAs, etc. through wired or wireless telecommunication.

The present invention uses specific numerical formulae in order to calculate physiological characteristics of lung-pulmonary circulation system using the primarily measured values as described above. Such numerical formulae mathematically illustrate physiological relations between breathing related values and, relate to the following variables and equations. Examples of input and output values include $O_2$-$CO_2$ information of mixed venous blood V*, $O_2$-$CO_2$ information of end-capillary C*, $O_2$-$CO_2$ information of arterial blood a*, $O_2$-$CO_2$ information of inspiration air I*, $O_2$-$CO_2$ information of alveolar gas A*, $O_2$-$CO_2$ information of end-tidal gas ET*, ventilation through alveolar VA, inspiration capacity VI, perfusion Q, cardiac output $Q_{total}$, shunt ratio Y, physiological dead space ratio X, etc. The equations include, for example: mass balance equations for $O_2$, $CO_2$ and $N_2$ such as Eq. 3 to 5; Fick's equations such as Eq. 1 and 2; shunt ratio equations such as Eq. 7 and 8; respiratory equations such as Eq. 13 and 14; ventilation-perfusion equations such as Eq. 9 to 12; cardiac output equations such as Eq. 15 and 16; and/or dead space ratio equations such as Eq. 17 and 18.

The present invention applies primary measurement values to be easily and non-invasively measured, for example, inspiration capacity, partial pressures of $O_2$ and $CO_2$ during ventilation and/or $O_2$ and $CO_2$ concentration measured in arterial blood in order to calculate or obtain important information such as: blood respiratory characteristics of lungs and pulmonary circulation organs, which have a significant difficulty in measurement; cardiac functional characteristics; and/or lung functional characteristics by adopting the following equations and values:

Fick's equations:

$$\dot{V}_{O2} = \dot{Q} \times (C_{C_{o_2}} - C_{\bar{V}_{o_2}}) \qquad (1)$$

$$\dot{V}_{CO2} = \dot{Q} \times (C_{\bar{V}_{co_2}} - C_{c_{co_2}}) \qquad (2)$$

Mass balance equations:

$$(\dot{V}_I/\dot{Q})P_{I_{o_2}} - (\dot{V}_A/\dot{Q})P_{A_{o_2}} = k \times (C_{c_{o_2}} - C_{\bar{V}_{o_2}}) \qquad (3)$$

$$(\dot{V}_A/\dot{Q})P_{A_{co_2}} = k \times (C_{\bar{V}_{co_2}} - C_{c_{o_2}}) \qquad (4)$$

$$(\dot{V}_I/\dot{Q})P_{I_{N_2}} - (\dot{V}_A/\dot{Q})P_{A_{N_2}} = \lambda \times (P_{c_{N_2}} - P_{\bar{V}_{N_2}}) \qquad (5)$$

$$P_{A_{o_2}} + P_{A_{co_2}} + P_{A_{N_2}} = P_B - P_{H2O} \qquad (6)$$

Shunt ratio equations:

$$Y_1 = \dot{Q}_S/\dot{Q}_T$$
$$= (C_{C'_{o_2}} - C_{a_{o_2}})/(C_{C'_{o_2}} - C_{\bar{V}_{o_2}}) \qquad (7)$$

$$Y_2 = \dot{Q}_S/\dot{Q}_T$$
$$= (C_{a_{co_2}} - C_{C'_{co_2}})/(C_{\bar{V}_{co_2}} - C_{C'_{co_2}}) \qquad (8)$$

Ventilation-perfusion ratio equations:

$$(\dot{V}_A/\dot{Q})_1 = \dot{V}_A \times (C_{c_{o_2}} - C_{\bar{V}_{o_2}})/\dot{V}_{O2} \qquad (9)$$

$$(\dot{V}_A/\dot{Q})_2 = \dot{V}_A \times (C_{\bar{V}_{co_2}} - C_{c_{co_2}})/\dot{V}_{CO2} \qquad (10)$$

$$( \dot{V}_A / \dot{Q})_3 = (( \dot{V}_I / \dot{Q}) P_{I_{O_2}} - k \times ( C_{C_{O_2}} - C_{\overline{V}_{O_2}}))/P_{A_{O_2}} \qquad (11)$$

$$( \dot{V}_A / \dot{Q})_4 = k \times ( C_{\overline{V}_{CO_2}} - C_{C_{O_2}})/P_{A_{CO_2}} \qquad (12)$$

Respiration quotient ratio equations:

$$R_1 = ( C_{\overline{V}_{CO_2}} - C_{C'_{CO_2}})/( C_{C'_{O_2}} - C_{\overline{V}_{O_2}}) \qquad (13)$$

$$R_2 = \frac{P_{A_{CO_2}}(1 - F_{I_{O_2}})}{P_{I_{O_2}} - F_{I_{O_2}} P_{A_{CO_2}} - P_{A_{O_2}}} = \frac{P_{ET_{CO_2}}(1 - F_{I_{O_2}})}{P_{I_{O_2}} - F_{I_{O_2}} P_{ET_{CO_2}} - P_{ET_{O_2}}} \qquad (14)$$

Cardiac output equations:

$$\dot{Q}_{total} = \dot{Q} / (1-Y) \qquad (15)$$

$$\dot{Q}_{total} = \dot{V}_I (1-X) / (1-Y) \qquad (16)$$

Dead space ratio equations:

$$X_1 = \dot{V}_D / \dot{V}_T$$
$$= ( P_{A_{O_2}} - P_{ET_{O_2}})/( P_{A_{O_2}} - P_{I_{O_2}}) \qquad (17)$$

$$X_2 = \dot{V}_D / \dot{V}_T$$
$$= ( P_{A_{CO_2}} - P_{ET_{CO_2}})/( P_{A_{CO_2}} - P_{I_{CO_2}}) \qquad (18)$$

Symbols used in the above equations have meanings as follows:

$W_{O2}$ :     oxygen uptake into capillary [ml/min]

$W_{CO2}$ :     carbon dioxide output into alveolar [ml/min]

$C_{CO2}$ :     $O_2$ concentration in capillary [%]

$C_{CCO2}$ :     $CO_2$ concentration in capillary [%]

$C_{\overline{VO2}}$:     $O_2$ concentration in mixed venous blood[%]

$C_{\overline{VCO2}}$:     $CO_2$ concentration in mixed venous blood[%]

$W$:     perfusion of capillary [liters/min]

$W_{total}$:     cardiac output [liters/min]

$P_{AO2}$:     $O_2$ partial pressure in alveolar [mmHg]

$P_{ACO2}$:     $CO_2$ partial pressure in alveolar [mmHg]

$P_{AN2}$:     partial $N_2$ pressure in alveolar [mmHg]

$P_{AH2O}$:     water vapor pressure in alveolar [mmHg]

$P_{IO2}$:     $O_2$ partial pressure of air in atmosphere [mmHg]

$P_{IN2}$:     partial $N_2$ pressure of air in atmosphere [mmHg]
$P_{CN2}$:     partial $N_2$ pressure in capillary [mmHg]
$P_{\overline{VN2}}$:     partial $N_2$ pressure in mixed venous blood[mmHg]
$W_I$:     flow rate of inspiration air [liters/min]
$W_A$:     flow rate of air gas-exchanged in alveolar [liters/min]
$W_D$:     flow rate of dead space air [liter/min]
$W_T$:     total inspiration or expiration capacity of air [liters/min]
X :     dead space ratio
Y :     shunt ratio
$\kappa$ :     respiratory quotient
$\lambda$ :     constant for blood and air
$Fi_{CO2}$ :     $CO_2$ partial pressure ratio in atmosphere
$Fi_{N2}$ :     partial $N_2$ pressure ratio in atmosphere
R :     respiratory quotient ratio (R = $W_{CO2}/W_{O2}$)

In order to verify the above methods and results thereof, a clinical respiratory data set M was prepared from five(5) individual patients with severe respiratory diseases hospitalized in the intensive care units (abbrev. to ICU patients) in Respiratory Medicine, Medicine College, Chung-Nam University of Korea. In the following Table 1, left two(2) columns showed $O_2$-$CO_2$ partial pressures of mixed venous blood V* among the prepared data set M. The measured data further comprises arterial blood data a*, inspiration data I* and data at the end of expiration ET*.

TABLE 1: $O_2$ and $CO_2$ concentrations of mixed venous blood

| Number of patients | Measured |
|---|---|
| | Mixed venous data A* (Pv$O_2$, Pv$CO_2$) [mmHg] |
| 1 | (42, 53) |
| 2 | (37, 63) |
| 3 | (36, 67) |
| 4 | (39, 42) |
| 5 | (53, 50) |

Alternatively, Fig. 7 illustrates $O_2$-$CO_2$ partial pressures of lung circulation system determined by the present invention, and corresponding values in the clinical measurement data set M of Chung-Nam University used as input values. Curves shown in Fig. 7 (1, 2, 3, 4 and 5) are ventilation-perfusion ratio curves resulted from solutions of 'first respiration model' questions for five patients. A symbol with a "diamond shape" at the left end of each of the curves (V1, V2 ... V5) represents measured $O_2$-$CO_2$ partial pressures value of mixed venous blood V* among the clinical data set M. A black circle (a1, a2 ... and a5) near the diamond symbol represents $O_2$-$CO_2$ partial pressures of arterial blood a* as clinical measurement value. A large triangle at center portion of each of the curves (A1 and A2 ... A5) is partial pressure A* of alveolar gas which was defined by using V*, a*, I* and ET* among the clinical measurement data set M as well as solutions of 'second respiration model' of the present invention. A blank square (E1, E2 ... E5) represents $CO_2$ partial pressure of end-tidal gas contained in the clinical measurement data set M marked on the ventilation-perfusion ratio curve obtained from 'first respiration model', while small and black squares near the blank square correctly indicate $O_2$-$CO_2$ partial pressures points of end-tidal gas ET* by further applying $O_2$ partial pressure of end-tidal gas calculated from solutions of 'second respiration model".

As shown in Fig. 7 and the above Table 1, when the calculated values were compared with the clinical values, it is clearly demonstrated that the systematic respiration analysis method according to the present invention can accurately predict and/or determine physiological characteristics of cardiopulmonary organs.

The present invention provides: a measurement apparatus to non-invasively determine partial $O_2$ and $CO_2$ pressures of ventilation gas and arterial blood, respectively; classification of two kinds of respiration models to sequentially solve and analyze complicated questions, so as to evaluate physiological characteristics such as blood respiratory characteristics of cardiopulmonary organs, cardiac functional characteristics, lung functional characteristics, etc.; and computer analysis of each of the respiration models and computing devices corresponding to the analysis. Especially, the present inventive measurement method has an advantage of improved accuracy and usefulness even for a lung with shunt and/or dead space as well as without the same.

In contrast to conventional techniques such as thermodilution that inserts a pulmonary arterial catheter into the pulmonary artery via the right ventricle and the right atrium, the present invention can eliminate pain, infection and/or complications of patients possibly caused by the catheter operation. Moreover, the present invention has no trouble of using electrodes required for receiving electrical bio-signals by attaching or fixing the electrodes to correct sites on hands (or arms) and legs (or feet). The present invention also considers equilibriums by $O_2$ diffusion as well as $CO_2$ diffusion in pulmonary capillary, compared to $CO_2$-rebreathing method developed by Novametrix which uses only $CO_2$ data obtained by breathing and is effective in a specific narrow range of cardiac outputs, so that various respiratory characteristics in lungs-pulmonary circulation system can be predicted or determined in the more extended range of cardiac outputs than that of the $CO_2$-rebreathing method.

While the present invention has been described with reference to the preferred embodiments, it will be understood by those skilled in the art that various modifications and variations may be made therein without departing from the scope of the present invention as defined by the appended claims.

## Claims

1.  A method for predicting respiratory characteristics, comprising the steps of:

    (a) inputting $O_2$-$CO_2$ partial pressure of mixed venous blood and $O_2$-$CO_2$ partial pressure of inspiration gas as specified boundary values, into an automatic computing device;
    (b) inputting an initial value VA/Q of ventilation-perfusion ratio for start of a do-loop into the automatic computing device;
    (c) inputting a pair of initial values of $O_2$-$CO_2$ partial pressure of alveolar gas, (A1, A2), for start of another do-loop, which satisfy ventilation-perfusion ratio equation, into the automatic computing device;
    (d) applying the $O_2$-$CO_2$ partial pressure of mixed venous blood, the $O_2$-$CO_2$ partial pressure of alveolar gas and the ventilation-perfusion ratio to solve the governing respiration equations;
    (e) solving a group of governing equations for respiratory blood gas in the automatic computing device and obtaining renewed values of alveolar-gas partial pressures (A1*, A2*) as a result;
    (f) calculating the renewed value VA/Q* for ventilation-perfusion ratio from the governing equations of respiratory gas, using the renewed $O_2$-$CO_2$ partial pressure of alveolar gas (A1*, A2*);
    (g) determining whether the renewed ventilation-perfusion ratio value VA/Q* satisfies the requirement of solution;
    (h) making decision whether the renewed $O_2$-$CO_2$ partial pressures (A1*, A2*) in pair with the renewed ventilation-perfusion ratio VA/Q* are correct solutions.

2.  The method according to claim 1, wherein the $O_2$-$CO_2$ partial pressures of mixed venous blood **V*** and the $O_2$-$CO_2$ partial pressures of inspiration gas **I*** in the step (a) are directly measured or usually obtained from alternative sources.

3.  The method according to claim 1, wherein the governing equations for respiratory blood gas in the step (e) comprises mass balance equations for $O_2$, $CO_2$ and $N_2$.

4.  The method according to claim 1, wherein the renewed ventilation-perfusion ratio VA/Q* in the step (f) is compared to the iterative initial value VA/Q in the step (b) to check whether the difference of VA/Q* and VA/Q is small enough relative to the value of VA/Q in the step (g).

5. The method according to claim 4, further comprising:

   calculation returning back to the steps (c) to (g) with renewed $O_2$-$CO_2$ partial pressures of alveolar gas, (A1, A2), if the ventilation-perfusion ratio VA/Q* does not satisfy requirement for solution in the step (g).

6. The method according to claim 5, wherein the renewed pair of $O_2$-$CO_2$ partial pressures of alveolar gas, (A1, A2), in the step (c) comprise $CO_2$ partial pressure, A2, that is renewed according to a specific regulation, and wherein the $O_2$ partial pressure, A1*, is renewed by the ventilation-perfusion ratio equation using the $CO_2$ partial pressure, A2.

7. The method according to claim 6, wherein the pair of $O_2$-$CO_2$ partial pressures of alveolar gas, (A1*, A2*), obtained in the step (e) comprise the renewed $O_2$ partial pressure, A1*, obtained in the step (c) and $CO_2$ partial pressure, A2*, obtained by solving the governing equations for respiratory blood gas using renewed $O_2$ partial pressure, A1*.

8. The method according to claim 5, wherein the renewed pair of $O_2$-$CO_2$ partial pressure of alveolar gas, (A1, A2), in the step (c) comprise $O_2$ partial pressure, A1, renewed according to a specific regulation, and wherein the renewed $CO_2$ partial pressure, A2*, obtained from the ventilation-perfusion ratio equation using $O_2$ partial pressure, A1.

9. The method according to claim 8, wherein the pair of $O_2$-$CO_2$ partial pressures of alveolar gas, (A1*, A2*), obtained in the step (e) comprise the renewed $CO_2$ partial pressure, A2*, obtained in the step (c) and $O_2$ partial pressure, A1*, obtained by solving the governing equations for respiratory blood gas using renewed $CO_2$ partial pressure, A2*.

10. The method according to any one of claims 6 to 9, wherein the $O_2$-$CO_2$ partial pressures of alveolar gas, (A1*, A2*), are obtained in the step (h) using initial ventilation-perfusion ratio VA/Q in the step (b), subsequent steps (c) to (e), and renewed ventilation-perfusion ratio VA/Q* in the step (f).

11. The method according to claim 10, wherein the step (h) includes: obtaining the solution of $O_2$-$CO_2$ partial pressures of alveolar gas, **A\*,** for the given initial ventilation-perfusion ratio VA/Q in the step (b); taking new initial value of VA/Q after returning back to the step (b); and repeating the steps (c) to (h).

12. The method according to claim 11, wherein the steps (a) to (h) are iterated over to obtain a set of alveolar $O_2$-$CO_2$ partial pressure solutions, corresponding to a set of pre-assigned initial values of the ventilation-perfusion ratio VA/Q.

13. A method for predicting respiratory characteristics, comprising the steps of:

   (a) inputting $O_2$-$CO_2$ partial pressures of mixed venous blood and $O_2$-$CO_2$ partial pressure of inspiration gas as specified boundary values, into an automatic computing device;
   (b) inputting a pair of initial values of $O_2$-$CO_2$ partial pressure of alveolar gas, (A1, A2), for start of a do-loop into the automatic computing device;
   (c) inputting an initial value VA/Q for ventilation-perfusion ratio, for start of another do-loop, which satisfy ventilation-perfusion ratio equation using the above $O_2$-$CO_2$ partial pressures initial values (A1, A2), into the automatic computing device;
   (d) applying the $O_2$-$CO_2$ partial pressure of mixed venous blood, the $O_2$-$CO_2$ partial pressure of alveolar gas and the ventilation-perfusion ratio to solve the governing respiration equations;
   (e) solving a group of governing equations for respiratory blood gas in the automatic computing device and obtaining renewed values of alveolar-gas partial pressures (A1*, A2*) as a result;
   (f) calculating the renewed value VA/Q* for ventilation-perfusion ratio from the governing equations of respiratory gas, using the renewed $O_2$-$CO_2$ partial pressure of alveolar gas (A1*, A2*);
   (g) determining whether the renewed $O_2$-$CO_2$ partial pressure of alveolar gas (A1*, A2*) satisfies requirement of solution; and
   (h) making decision whether the renewed $O_2$-$CO_2$ partial pressures (A1*, A2*) in pair with the initial ventilation-perfusion ratio VA/Q are correct solutions.

14. The method according to claim 13, wherein the $O_2$-$CO_2$ partial pressures of mixed venous blood V* and the $O_2$-$CO_2$ partial pressures of inspiration gas I* in the step (a) are directly measured or usually obtained from alternative sources.

**15.** The method according to claim 13, wherein the governing equations for respiratory blood gas in the step (e) comprises mass balance equations for $O_2$, $CO_2$ and $N_2$.

**16.** The method according to claim 13, wherein the renewed ventilation-perfusion ratio VA/Q* in the step (f) is compared to the iterative initial value VA/Q in the step (b) to check whether the difference of VA/Q* and VA/Q is small enough relative to the value of VA/Q in the step (g).

**17.** The method according to claim 16, further comprising: calculation returning back to the steps (c) to (g) with renewed $O_2$-$CO_2$ partial pressures of alveolar gas, (A1, A2), if the ventilation-perfusion ratio VA/Q* does not satisfy requirement for solution in the step (g).

**18.** The method according to claim 17, wherein the renewed pair of $O_2$-$CO_2$ partial pressure of alveolar gas, (A1, A2), in the step (b) comprise $CO_2$ partial pressure, A2, setup as an initial value and $O_2$ partial pressure A1 obtained by mass balance equation for $O_2$ using the $CO_2$ partial pressure A2.

**19.** The method according to claim 18, wherein the pair of $O_2$-$CO_2$ partial pressure of alveolar gas (A1*, A2*) obtained in the step (e) comprise the initial $CO_2$ partial pressure A2 obtained in the step (b) and $O_2$ partial pressure A1* obtained by solving a group of governing equations for respiratory blood gas using the initial $CO_2$ partial pressure A2.

**20.** The method according to claim 17, wherein the renewed pair of $O_2$-$CO_2$ partial pressure of alveolar gas (A1, A2) in the step (b) comprise $O_2$ partial pressure A1 setup as an initial value and $CO_2$ partial pressure A2 obtained by mass balance equation for $CO_2$ using the $O_2$ partial pressure A1.

**21.** The method according to claim 20, wherein the pair of $O_2$-$CO_2$ partial pressure of alveolar gas (A1*, A2*) obtained in the step (e) comprise the initial $O_2$ partial pressure A1 obtained in the step (b) and $CO_2$ partial pressure A2* obtained by solving a group of governing equations for respiratory blood gas using the initial $O_2$ partial pressure A1.

**22.** The method according to any one of claims 18 to 21, wherein the pair of $O_2$-$CO_2$ partial pressure of alveolar gas (A1, A2) satisfying the ventilation-perfusion ratio requirement in the step (h) comprise solutions (A1*, A2*) satisfying the equation for respiratory blood gas using initial value VA/Q for ventilation-perfusion ratio.

**23.** The method according to claim 22, further comprising the steps of: obtaining final solutions of $O_2$-$CO_2$ partial pressure of alveolar gas (A1*, A2*) with regard to $O_2$-$CO_2$ partial pressures initial values of alveolar gas (A1, A2) or initial value VA/Q for ventilation-perfusion ratio corresponding thereto; renewing $O_2$ partial pressure A1 after returning to the step (b); and repeatedly carrying out the steps (c) to (h).

**24.** The method according to claim 22, further comprising the steps of: obtaining final solutions of $O_2$-$CO_2$ partial pressure of alveolar gas (A1*, A2*) with regard to $O_2$-$CO_2$ partial pressures initial values of alveolar gas (A1, A2) or initial value VA/Q for ventilation-perfusion ratio corresponding thereto; renewing $CO_2$ partial pressure A2 after returning to the step (b); and repeatedly carrying out the steps (c) to (h).

**25.** A method for predicting respiratory characteristics, comprising the steps of:

(a) inputting blood boundary value, gas boundary value, supporting information for blood, supporting information for gas and inspiration flow rate into an automatic computing device;
(b) inputting initial value of physiological dead space ratio X into the automatic computing device;
(c) inputting a pair of $O_2$-$CO_2$ partial pressures initial values of alveolar gas (A1, A2) into the automatic computing device using the initial value of the dead space ratio X;
(d) applying the boundary value, the initial value and the initial values to an computing subroutines built in the automatic computing device;
(e) solving a group of governing equations for respiratory blood gas in the computing subroutines and obtaining newly renewed $O_2$-$CO_2$ partial pressure of alveolar gas (A1*, A2*) based on the solutions;
(f) calculating $O_2$ shunt ratio Y1 and $CO_2$ shunt ratio Y2 if the renewed $O_2$-$CO_2$ partial pressure satisfy requirement for $O_2$-$CO_2$ partial pressures;
(g) determining desired respiratory characteristics if the shunt ratio requirement is satisfied; and
(h) determining cardiac output.

**26.** The method according to claim 25, wherein the blood boundary value in the step (a) comprises $O_2$-$CO_2$ partial

pressures V* of mixed venous blood or is obtained from alternative sources.

27. The method according to claim 25, wherein the gas boundary value in the step (a) comprises all of $O_2$-$CO_2$ partial pressure at inspiration I* or $O_2$ partial pressure only.

28. The method according to claim 25, wherein the supporting information for blood in the step (a) comprises all of $O_2$-$CO_2$ partial pressure of arterial blood a* or $O_2$ partial pressure only.

29. The method according to claim 25, wherein the supporting information for gas in the step (a) comprises all of $O_2$-$CO_2$ partial pressure of end-tidal gas ET* or $CO_2$ partial pressure only.

30. The method according to claim 25, wherein the inspiration capacity VI in the step (a) means flow rate of external air entered into lungs and, for tidal breathing, the capacity VI is substantially equal to expiration capacity VE released outside from the lungs.

31. The method according to claim 25, wherein the respiratory governing equations for respiratory blood gas in the step (e) comprises mass balance equations for $O_2$, $CO_2$ and $N_2$ and combined equations for gas partial pressure.

32. The method according to claim 25, further comprising: repetition of the steps (d) and (e) for a renewed pair of $O_2$-$CO_2$ partial pressures initial values of alveolar gas (A1, A2) after returning to the step (c) if the requirement for $O_2$-$CO_2$ partial pressures in the step (f) was not satisfied.

33. The method according to claim 32, wherein the $O_2$-$CO_2$ partial pressure (A1, A2) in the step (c) are a new pair of partial pressures (A1, A2*) comprising $O_2$ partial pressure A1 to be a repeatedly renewed initial value and $CO_2$ partial pressure A2* obtained using the dead space ratio X.

34. The method according to claim 33, wherein the $O_2$-$CO_2$ partial pressure of alveolar gas (A1*, A2*) obtained in the step (e) comprise the $CO_2$ partial pressure of alveolar gas A2* obtained in the step (c) and renewed $O_2$ partial pressure A1* obtained by solving a group of governing equations for respiratory blood gas including mass balance equations for $O_2$, $CO_2$ and $N_2$ and combined equations for gas partial pressure using the $O_2$-$CO_2$ partial pressure (A1, A2*) determined in the step (c).

35. The method according to claim 34, wherein the requirement for $O_2$-$CO_2$ partial pressures in the step (f) is **characterized in** determining whether a difference between the $O_2$ partial pressure A1 renewed initial value in the step (c) and the $O_2$ partial pressure A1* obtained by solving a group of governing equations for respiratory blood gas is within a specific range.

36. The method according to claim 32, wherein the $O_2$-$CO_2$ partial pressure (A1, A2) in the step (c) are a new pair of partial pressures (A1*, A2) comprising $CO_2$ partial pressure A2 to be a repeatedly renewed initial value and $O_2$ partial pressure A1* obtained using the initial value of the dead space ratio X.

37. The method according to claim 36, wherein the pair of $O_2$-$CO_2$ partial pressure of alveolar gas (A1*, A2*) obtained in the step (e) comprise the $O_2$ partial pressure of alveolar gas A1* obtained in the step (c) and renewed $CO_2$ partial pressure A2* obtained by solving a group of governing equations for respiratory blood gas including mass balance equations for $O_2$, $CO_2$ and $N_2$ and combined equations for gas partial pressure using the $O_2$-$CO_2$ partial pressure (A1*, A2) determined in the step (c).

38. The method according to claim 37, wherein requirement for $O_2$-$CO_2$ partial pressures in the step (f) is **characterized in** determining whether a difference between the $CO_2$ partial pressure A2 renewed initial value in the step (c) and the $CO_2$ partial pressure A2* obtained by solving a group of governing equations for respiratory blood gas is within a specific range.

39. The method according to claim 25, wherein requirement for shunt ratio in the step (g) is **characterized in** determining whether a difference between $O_2$ shunt ratio Y1 and $CO_2$ shunt ratio Y2 is within a specific range.

40. The method according to claim 25, further comprising: returning to the step (b) and repeatedly renewing physiological dead space ratio X, if the requirement for dead space ratio in the step (g) is not satisfied.

41. The method according to claim 25, wherein respiratory characteristics determined in the step (g) includes any one selected from $O_2$-$CO_2$ partial pressures A* of alveolar gas, $O_2$-$CO_2$ partial pressures of capillary C*, shunt ratio Y* and physiological dead space ratio X*.

42. The method according to claim 25, wherein cardiac output determined in the step (h) is obtained by using measured inspiration capacity VI or expiration capacity VE and physiological dead space ratio X*.

43. A method for predicting respiratory characteristics, comprising the steps of:

> (a) inputting blood boundary value, gas boundary value, supporting information for blood, supporting information for gas and inspiration capacity into an automatic computing device;
> (b) inputting an initial value of shunt ratio Y into the automatic computing device;
> (c) inputting a pair of $O_2$-$CO_2$ partial pressures initial values of alveolar gas (A1, A2) obtained by the initial shunt ratio Y into the automatic computing device;
> (d) applying the boundary value, the initial value and the initial values to computing subroutines built in the automatic computing device;
> (e) solving a group of governing equations for respiratory blood gas in the computing subroutines and obtaining renewed $O_2$-$CO_2$ partial pressure of alveolar gas (A1*, A2*) based on the solutions of the equation group;
> (f) calculating $O_2$ dead space ratio X1 and $CO_2$ dead space ratio X2 if the renewed $O_2$-$CO_2$ partial pressure (A1*, A2*) satisfy requirements for $O_2$-$CO_2$ partial pressures;
> (g) determining desired respiratory characteristics if the dead space ratio requirement is satisfied; and
> (h) determining cardiac output.

44. The method according to claim 43, wherein the blood boundary value in the step (a) comprises $O_2$-$CO_2$ partial pressures V* of mixed venous blood or is obtained from alternative sources.

45. The method according to claim 43, wherein the gas boundary value in the step (a) comprises all of $O_2$-$CO_2$ partial pressure at inspiration I* or $O_2$ partial pressure only.

46. The method according to claim 43, wherein the supporting information for blood in the step (a) comprises all of $O_2$-$CO_2$ partial pressure of arterial blood a* or $O_2$ partial pressure only.

47. The method according to claim 43, wherein the supporting information for gas in the step (a) comprises all of $O_2$-$CO_2$ partial pressure of end-tidal gas ET* or $CO_2$ partial pressure only.

48. The method according to claim 43, wherein the inspiration capacity VI in the step (a) means flow rate of external air entered into lungs and is substantially equal to expiration capacity VE released outside from the lungs.

49. The method according to claim 43, wherein the respiratory governing equations for respiratory blood gas in the step (e) comprises mass balance equations for $O_2$, $CO_2$ and $N_2$ and combined equations for gas partial pressure.

50. The method according to claim 43, further comprising: repetition of the steps (d) and (e) for a renewed pair of $O_2$-$CO_2$ partial pressures initial values of alveolar gas (A1, A2) after returning to the step (c) if the requirement for $O_2$-$CO_2$ partial pressures in the step (f) was not satisfied.

51. The method according to claim 50, wherein the $O_2$-$CO_2$ partial pressure (A1, A2) in the step (c) are a new pair of partial pressures (A1, A2*) comprising $O_2$ partial pressure A1 to be a repeatedly renewed initial value and $CO_2$ partial pressure A2* obtained using the initial shunt ratio Y.

52. The method according to claim 50, wherein the renewed $O_2$-$CO_2$ partial pressure of alveolar gas (A1*, A2*) obtained in the step (e) comprise the $CO_2$ partial pressure of alveolar gas A2* obtained in the step (c) and renewed $O_2$ partial pressure A1* obtained by solving a group of governing equations for respiratory blood gas including mass balance equations for $O_2$, $CO_2$ and $N_2$ and combined equations for gas partial pressure using the $O_2$-$CO_2$ partial pressure (A1, A2*) determined in the step (c).

53. The method according to claim 52, wherein the requirement for $O_2$-$CO_2$ partial pressures in the step (f) is **characterized in** determining whether a difference between the $O_2$ partial pressure A1 renewed initial value in the step (c) and the $O_2$ partial pressure A1* obtained by solving a group of governing equations for respiratory blood gas is within a specific range.

54. The method according to claim 50, wherein the pair of $O_2$-$CO_2$ partial pressure (A1, A2) in the step (c) are a new pair of partial pressures (A1*, A2) comprising $CO_2$ partial pressure A2 to be a repeatedly renewed initial value and $O_2$ partial pressure A1* obtained using the initial shunt ratio Y.

55. The method according to claim 54, wherein the renewed $O_2$-$CO_2$ partial pressure of alveolar gas (A1*, A2*) obtained in the step (e) comprise the $O_2$ partial pressure of alveolar gas A1* obtained in the step (c) and renewed $CO_2$ partial pressure A2* obtained by solving a group of governing equations for respiratory blood gas including mass balance equations for $O_2$, $CO_2$ and $N_2$ and combined equations for gas partial pressure using the $O_2$-$CO_2$ partial pressure (A1*, A2) determined in the step (c).

56. The method according to claim 55, wherein requirement for $O_2$-$CO_2$ partial pressures in the step (f) is **characterized in** determining whether a difference between the $CO_2$ partial pressure A2 renewed initial value in the step (c) and the $CO_2$ partial pressure A2* obtained by solving a group of governing equations for respiratory blood gas is within a specific range.

57. The method according to claim 43, wherein requirement for shunt ratio in the step (g) is **characterized in** determining whether a difference between $O_2$ dead space ratio X1 and $CO_2$ dead space ratio X2 is within a specific range, and which comprises returning to the step (b) and repeatedly renewing physiological shunt ratio Y, if the requirement for dead space ratio is not satisfied.

58. The method according to claim 43, further comprising: returning to the step (b) and repeatedly renewing physiological shunt ratio Y, if the requirement for dead space ratio in the step (g) is not satisfied.

59. The method according to claim 43, wherein respiratory characteristics determined in the step (g) includes any one selected from $O_2$-$CO_2$ partial pressures A* of alveolar gas, $O_2$-$CO_2$ partial pressures of capillary C*, shunt ratio Y* and physiological dead space ratio X*.

60. The method according to claim 43, wherein cardiac output determined in the step (h) is obtained by using measured inspiration capacity VI or expiration capacity VE and physiological dead space ratio X*.

61. An instrument for displaying respiratory characteristics including an information terminal connected to an automatic computing device to visually display respiratory characteristics which are predicted or determined by a method for determining the respiratory characteristics defined in any one of claims 1, 13, 25 and 43.

62. The instrument according to claim 61, wherein the information terminal is wired or wirelessly connected to the automatic computing device and portably carried.

63. The instrument according to claim 62, wherein the automatic computing device comprises a computer processor or embedded chip.

Fig. 1

Fig. 2

Fig. 3

Fig. 4

Fig. 5

Fig. 6

Fig. 7

**European Patent Office**

## EUROPEAN SEARCH REPORT

Application Number

EP 08 00 4086

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 258 038 B1 (HARYADI DINESH G [IN] ET AL) 10 July 2001 (2001-07-10) | 1,13,25, 43 | INV. A61B5/083 |
| X | * column 6, line 55 - column 14, line 29 * ----- | 61 | |
| A | US 2001/029339 A1 (ORR JOSEPH A [US] ET AL ORR JOSEPH A [US] ET AL) 11 October 2001 (2001-10-11) * paragraphs [0071] - [0097] * ----- | 1,13,25, 43,61 | |
| A | US 7 008 380 B1 (REES STEPHEN EDWARD [DK] ET AL) 7 March 2006 (2006-03-07) * the whole document * ----- | 1,13,25, 43,61 | |

TECHNICAL FIELDS SEARCHED (IPC)

A61B

The present search report has been drawn up for all claims

| Place of search | Date of completion of the search | Examiner |
|---|---|---|
| Munich | 13 May 2008 | Manschot, Jan |

EPO FORM 1503 03.82 (P04C01)

## ANNEX TO THE EUROPEAN SEARCH REPORT
## ON EUROPEAN PATENT APPLICATION NO.

EP 08 00 4086

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2008

| Patent document cited in search report | | Publication date | Patent family member(s) | | Publication date |
|---|---|---|---|---|---|
| US 6258038 | B1 | 10-07-2001 | WO | 0013581 A1 | 16-03-2000 |
| | | | US | 6042550 A | 28-03-2000 |
| | | | US | 6241681 B1 | 05-06-2001 |
| US 2001029339 | A1 | 11-10-2001 | BR | 9714427 A | 25-04-2000 |
| | | | EP | 0999783 A1 | 17-05-2000 |
| | | | JP | 3521915 B2 | 26-04-2004 |
| | | | JP | 2001506158 T | 15-05-2001 |
| | | | JP | 3955011 B2 | 08-08-2007 |
| | | | JP | 2005152001 A | 16-06-2005 |
| | | | WO | 9826710 A1 | 25-06-1998 |
| | | | US | 2005203432 A1 | 15-09-2005 |
| | | | US | 6227196 B1 | 08-05-2001 |
| | | | US | 2006129054 A1 | 15-06-2006 |
| | | | US | 2004059239 A1 | 25-03-2004 |
| | | | US | 2004049113 A1 | 11-03-2004 |
| | | | US | 2001031928 A1 | 18-10-2001 |
| | | | US | 6306098 B1 | 23-10-2001 |
| US 7008380 | B1 | 07-03-2006 | AU | 774661 B2 | 01-07-2004 |
| | | | AU | 2278800 A | 25-08-2000 |
| | | | CA | 2359575 A1 | 10-08-2000 |
| | | | WO | 0045702 A1 | 10-08-2000 |
| | | | EP | 1152690 A1 | 14-11-2001 |
| | | | JP | 2002536039 T | 29-10-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020070021264
- KR 19870002027
- KR 1019990000417
- KR 199922493